# EUROPÄISCHE PATENTSCHRIFT

(11) **EP 3 457 910 B1**
(45) Veröffentlichungstag und Bekanntmachung des Hinweises auf die Patenterteilung: **25.08.2021**
(21) Anmeldenummer: 17721676.9
(22) Anmeldetag: 05.05.2017
(51) Int. Cl.: A61B 5/00

(54) **VERFAHREN UND VORRICHTUNG ZUM ERMITTELN EINES SCHÄDIGUNGSGRADS VON HAAR**
METHOD AND DEVICE FOR DETERMINING A DEGREE OF DAMAGE TO HAIR
PROCÉDÉ ET DISPOSITIF PERMETTANT DE DÉTERMINER UN DEGRÉ DE DÉTÉRIORATION DES CHEVEUX

(30) Priorität: 19.05.2016 DE 102016208631
(43) Veröffentlichungstag der Anmeldung: 27.03.2019
(73) Patentinhaber: Henkel AG & Co. KGaA, 40589 Düsseldorf (DE)
(72) Erfinder: KNÜBEL, Georg, 40219 Düsseldorf (DE); GEBERT-SCHWARZWÄLDER, Antje, 41469 Neuss (DE); KOENEN, Annika, 41516 Grevenbroich (DE); KROOS, Astrid, 40789 Monheim (DE); MOCH, Melanie, 41542 Dormagen (DE); HOWORKA, Wilfried, 40593 Düsseldorf (DE)
(86) Internationale Anmeldenummer: PCT/EP2017/060758
(87) Internationale Veröffentlichungsnummer: WO 2017/198479

(56) Entgegenhaltungen:
- EP-A1- 1 877 027
- JP-A- H09 127 105
- US-A1- 2008 161 661
- US-A1- 2011 155 161
- US-A1- 2015 148 684
- US-A1- 2015 223 749
- US-A1- 2016 058 689
- SANDHU S S ET AL: "A SENSITIVE FLUORESCENCE TECHNIQUE USING DANSYL CHLORIDE TO ASSESS HAIR DAMAGE", JOURNAL OF THE SOCIETY COSMETIC CHEMI, SOCIETY OF COSMETIC CHEMISTS, US, Bd. 40, Nr. 5, 1. September 1989 (1989-09-01), Seiten 287-296, XP009144064, ISSN: 0037-9832
- M L Tate ET AL: "Quantification and prevention of hair damage", j. Soc. Cosmet. Chem, 1 November 1993 (1993-11-01), pages 347-371, XP055513866, Retrieved from the Internet: URL:http://citeseerx.ist.psu.edu/viewdoc/d ownload?doi=10.1.1.471.2274&rep=rep1&type= pdf
- DOS SANTOS SILVA A L ET AL: "Rhodamine B diffusion in hair as a probe for structural integrity", COLLOIDS AND SURFACES. B, BIOINTERFACES, ELSEVIER, AMSTERDAM, NL, vol. 40, no. 1, 15 January 2005 (2005-01-15), pages 19-24, XP027803318, ISSN: 0927-7765 [retrieved on 2005-01-15]
- Alexander Ehlers ET AL: "Multiphoton fluorescence lifetime imaging of human hair", MICROSCOPY RESEARCH AND TECHNIQUE., vol. 70, no. 2, 1 February 2007 (2007-02-01), pages 154-161, XP055638624, GB ISSN: 1059-910X, DOI: 10.1002/jemt.20395

## Beschreibung

Die Erfindung betrifft ein Verfahren und Vorrichtung zum Ermitteln eines Schädigungsgrads von Haar und Verfahren zum Ermitteln eines nutzerspezifischen Mittels zur Haarbehandlung.

Bei einer Behandlung von Haar mit kosmetischen Produkten kann eine Wirkung des Produkts, z.B. eine Intensität einer Färbung, stark vom Schädigungsgrad des Haars abhängen.

Deshalb kann eine Ermittlung einer Schädigung des Haars von großer Bedeutung sein.

Haar kann durch natürliche oder künstlich herbeigeführte Vorgänge geschädigt werden. Der wichtigste Schädigungstyp kann dabei eine oxidative Schädigung sein.

Die natürlichen Vorgänge können beispielsweise eine kombinierte (z.B. gleichzeitige) Einwirkung von UV-Licht und Sauerstoff (O₂) auf das Haar aufweisen.

Die künstlich herbeigeführten Vorgänge können dabei beispielsweise ein Anwenden von Haarfärbemitteln (auch als Colorationen bezeichnet), ein Blondieren, und/oder ein Erzeugen einer Dauerwelle aufweisen.

Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Der Schädigungsvorgang kann dabei ursächlich durch eine Oxidation von Aminosäuren, beispielsweise eine Oxidation der im Haar sehr häufig vorkommenden Aminosäuren Cystin und Cystein zu Cysteinsäure, erfolgen. Cystin kann im Haar intermolekulare Disulfidbrücken (auch als S-S-Brücken bezeichnet) ausbilden, so dass das Cystin äußerst wichtig ist für die mechanische Stabilität des Haars.

Die Oxidation dieser Brücken zu Cysteinsäure kann die mechanische Stabilität des Haars zerstören und bei mehrfachen Anwendungen sogar zu einem vollständigen Haarbruch führen. Allerdings können bereits vorher makroskopisch wahrnehmbare, z.B. fühlbare, Eigenschaften des Haars, beispielsweise eine Oberflächenrauhigkeit, negativ beeinflusst werden. Auch Ergebnisse kosmetischer Behandlungen, insbesondere schädigender Verfahren, können bereits in einem frühen Schädigungsstadium massiv verändert sein im Vergleich zu einem Ergebnis bei ungeschädigtem Haar.
Der beschriebene Schädigungsmechanismus ermöglicht es, den Grad der wichtigsten, nämlich der oxidativen Schädigung, durch eine Bestimmung eines Gehaltes an Cysteinsäure exakt zu ermitteln. In einem akademischen und industriellen Bereich können einem Forscher bzw. Entwickler eine Vielzahl von physikalischen und chemisch-analytischen Verfahren zur Verfügung stehen, um eine Ermittlung des Schädigungsgrads durchzuführen, beispielsweise eine quantitative Ermittlung des oxidativen Schädigungsgrads.
Herkömmlich werden hierbei chromatographische Verfahren verwendet, wie beispielsweise Hochleistungsflüssigkeitschromatographie (HPLC) nach aufwändiger Hydrolyse.
Dokument US 2011155161 A1 beschreibt ein Verfahren zur kosmetischen Behandlung von Haaren, das einen Schritt zum Nachweis eines vom Haar emittierten Fluoreszenzsignals umfasst. Dokument EP1 877 027 A1 beschreibt ein Öl-in-Glykol-Gel, das zur Reparatur von tiefen und oberflächlichen Schäden im Haar verwendet werden kann.
Ungeschädigtes Haar kann typischerweise einen Cysteinsäuregehalt in einem Bereich von etwa 0.5% bis etwa 1% (nach Gewicht) aufweisen. Bei Vorliegen einer Schädigung, beispielsweise infolge mehrfachen Ultrablondierens und/oder anderer Schädigungsmechanismen, kann der Cysteinsäuregehalt auf über 15% (Gew.) ansteigen.
Allerdings sind all diese chromatographischen Verfahren kompliziert und apparativ aufwändig, so dass sie einem Endverbraucher nicht zur Verfügung stehen.
Schädigende kosmetische Behandlungen, beispielsweise Hitzeanwendungen, Dauerwellen oder oxidative Prozeduren wie z.B. Blondieren, und viele andere mehr, werden typischerweise im privaten Bereich oder im Bereich kommerzieller Dienstleistungen am Endverbraucher ausgeführt. Obwohl eine Durchführung eines weiteren schädigenden Verfahrens an vorgeschädigtem Haar zu katastrophalen Ergebnissen bis hin zu einem vollständigen Haarbruch führen kann, bestand in diesem Rahmen bisher keine Möglichkeit, den Grad der Vorschädigung des Haars, beispielsweise quantitativ, zu bestimmen.
Eine Mehrzahl von Fluoreszenzfarbstoffen wird von oxidativ geschädigtem Haar stärker adsorbiert als von ungeschädigtem Haar. Ursache hierfür kann eine starke Hydrophilie einer Haaroberfläche nach einer oxidativen Schädigung im Gegensatz zu einer ausgeprägten Hydrophobie von ungeschädigtem Haar sein.
In verschiedenen Ausführungsbeispielen wird diese Eigenschaft genutzt, um ein Verfahren bereitzustellen, einen Schädigungsgrad von Haar durch eine Quantifizierung, beispielsweise ausgedrückt als ein Gehalt an Cysteinsäure, einer Fluoreszenzintensität der (geschädigten) Haarfläche zu ermitteln.

In verschiedenen Ausführungsbeispielen zeigt geschädigtes Haar eine Eigenfluoreszenz, welche genutzt wird, um ein Verfahren bereitzustellen, einen Schädigungsgrad von Haar durch eine Quantifizierung, beispielsweise ausgedrückt als ein Gehalt an Cysteinsäure, einer Fluoreszenzintensität der (geschädigten) Haarfläche zu ermitteln.

In verschiedenen Ausführungsbeispielen können für die Quantifizierung geeignete mathematische Modelle der Prädiktiven Analytik genutzt werden.

In verschiedenen Ausführungsbeispielen wird ein in einer Nutzung einfaches Verfahren bereitgestellt, welches mit Hilfe von Fluoreszenz-Detektion und Methoden aus der Prädiktiven Analytik eine präzise Ermittlung eines Grads einer oxidativen Schädigung von Haar ermöglicht.

In verschiedenen Ausführungsbeispielen kann das Verfahren aufgrund seiner einfachen experimentellen Durchführbarkeit geeignet sein für eine Ausführung unter Verwendung einer mobilen Datenverarbeitungsvorrichtung. Als mobile Datenverarbeitungsvorrichtung kann dabei beispielsweise ein Smartphone oder ein Tablet oder Laptop genutzt werden.

In verschiedenen Ausführungsbeispielen kann ein Verfahren bereitgestellt werden, welches es ermöglicht, mittels einfacher chemischer und bildanalytischer Verfahren, welche sich beispielsweise unter Verwendung einer mobilen Datenverarbeitungsvorrichtung (z.B. eines Smartphones), weniger weiterer einfacher Vorrichtungen (z.B. einer UV-LED) und eines Prädiktive-Analytik-Verfahrens verwirklichen lassen, einen Schädigungsgrad von Haar exakt zu bestimmen. Dies kann eine gezielte Auswahl von Produkten, z.B. kosmetischen Produkten, und/oder ein Bereitstellen personalisierter kosmetischer Produkte, z.B. Haarkosmetika, ermöglichen.

In verschiedenen Ausführungsbeispielen kann das Verfahren zum Ermitteln des Schädigungsgrads von Haar verwendet werden in einem Verfahren zum Auswählen von geeigneten, d.h. zum Erzielen einer gewünschten Wirkung geeigneten, kosmetischen Produkten. Die geeigneten Produkte können dabei gemäß verschiedenen Ausführungsbeispielen auch personalisierte (so genannte "masscustomized") Kosmetika aufweisen.

In verschiedenen Ausführungsbeispielen kann eine Intensität von Fluoreszenzlicht mit Hilfe von bildanalytischen Verfahren unter normierten Bedingungen einfach erfasst werden. Durch eine Anwendung von mathematischen Modellen aus dem Bereich Prädiktive Analytik kann mittels Vermessung von Standard-Haarproben, welche einen anhand bekannter aufwändiger Verfahren ermittelten Cysteinsäuregehalt aufweisen, ein mathematisches Modell erstellt werden, welches dann bei einer Haarprobe, auch als Tresse bezeichnet, des Verbrauchers anhand des registrierten Fluoreszenzichts und der daraus ermittelten Fluoreszenzintensität eine Berechnung eines Gehalts an Cysteinsäure, und damit der Haarschädigung, erlaubt. Die Bildanalyse kann dabei beispielsweise (mit geeigneten Apps) mittels bekannter Smartphones, Tablets o.ä. ausgeführt werden.

In verschiedenen Ausführungsbeispielen erlaubt die Verwendung der mathematischen Modelle aus dem Bereich Prädiktive Analytik (wie z.B. Tree Ensembles, neuronale Netze oder Support Vector Machines) eine wesentlich exaktere Berechnung der Schädigung (welche in den Modellen eine abhängige Variable bildet), als dies mit einfachen Modellen, z.B. einer einfachen linearen Regression, möglich wäre. Die Verfahren können dabei eine Vielzahl von Input-Variablen parallel nutzen und auch nicht-lineare Zusammenhänge abbilden. In verschiedenen Ausführungsbeispielen ermöglichen diese Modelle beispielsweise eine Einbeziehung kategorialer, nicht-metrischer Input-Variablen, wie z.B. einer Haarfarbe (z.B. blond, braun, schwarz, usw.) und/oder ethnischer Zugehörigkeit eines Haartyps (z.B. kaukasisch, asiatisch, afro-amerikanisch), welche einen Einfluss auf eine resultierende Fluoreszenzintensität haben können.

Die Haarfarbe kann alternativ auch als metrische Variable in das Modell eingehen.

In verschiedenen Ausführungsbeispielen wird ein Verfahren zum Ermitteln eines Schädigungsgrads von Haar bereitgestellt. Das Verfahren kann aufweisen: während eines Belichtens einer Haarprobe mit UV-Licht, Registrieren von Fluoreszenzlicht, welches von der Haarprobe emittiert wird, Ermitteln einer Fluoreszenzintensität anhand des registrierten Lichts, und Ermitteln des Schädigungsgrads des Haars unter Einbeziehung der Fluoreszenzintensität der Haarprobe.

In verschiedenen Ausführungsbeispielen wird ein Verfahren zum Ermitteln eines Schädigungsgrads von Haar bereitgestellt. Das Verfahren kann aufweisen: Benetzen einer dem Haar entnommenen Haarprobe mit einer Fluoreszenzfarbstofflösung, Entfernen von nicht in der Haarprobe adsorbierter Fluoreszenzfarbstofflösung, während eines Belichtens des Haars mit UV-Licht, Registrieren von Licht, welches von in der Haarprobe adsorbiertem Fluoreszenzfarbstoff emittiert wird, Ermitteln einer Fluoreszenzintensität anhand des registrierten Lichts, und Ermitteln des Schädigungsgrads des Haars unter Einbeziehung der Fluoreszenzintensität der Haarprobe.

Anders ausgedrückt kann in verschiedenen Ausführungsbeispielen die Eigenfluoreszenz des geschädigten Haars, die Fluoreszenz von zusätzlich in die Haarprobe eingebrachten Fluoreszenzfarbstoffen oder beides bei einem Verfahren zum Ermitteln des Schädigungsgrads des Haars genutzt werden.

Gemäß verschiedenen Ausführungsformen kann das Ermitteln des Schädigungsgrads ein Ermitteln einer Beziehung zwischen einer Mehrzahl von fluoreszenzintensitätbeeinflussenden Parametern und der Fluoreszenzintensität mittels prädiktiver Analytik aufweisen.

Gemäß verschiedenen Ausführungsformen kann die Mehrzahl von fluoreszenzintensitätbeeinflussenden Parametern eine Haarfarbe und/oder eine ethnische Zugehörigkeit eines Typs des Haars aufweisen.

Gemäß verschiedenen Ausführungsformen kann die prädiktive Analytik mindestens ein Verfahren nutzen aus einer Gruppe von Verfahren, wobei die Gruppe von Verfahren aufweist:
lineare oder multi-lineare Regression, polynome Regression, neuronale-Netze-Verfahren, Support Vector Machine-Verfahren, Entscheidungsbäume-Verfahren ("Decision Trees", "Random Forest", "Tree Ensembles") und weitere Verfahren.

Gemäß verschiedenen Ausführungsformen kann das Registrieren von Licht ein Erzeugen eines Fotos aufweisen.

Gemäß verschiedenen Ausführungsformen kann das Registrieren von Licht ein direktes Registrieren des Fluoreszenzlichts aufweisen.

Gemäß verschiedenen Ausführungsformen kann das Registrieren von Licht, das Ermitteln einer Fluoreszenzintensität und/oder das Ermitteln des Schädigungsgrads des Haars mittels einer mobilen Datenverarbeitungsvorrichtung ausgeführt werden.

Gemäß verschiedenen Ausführungsformen kann die Fluoreszenzfarbstofflösung Rhodamin B, Cumarin und/oder Fluorescein aufweisen.

Gemäß verschiedenen Ausführungsformen kann auf ein Benetzen des Haars mit der Fluoreszenzfarbstofflösung verzichtet werden und anstelle der vom geschädigten Haar aus der Fluoreszenzfarbstofflösung aufgenommenen Fluoreszenzfarbstoffe die Eigenfluoreszenz des geschädigten Haars ermittelt werden.

Gemäß verschiedenen Ausführungsformen kann das UV-Licht eine Wellenlänge in einem Bereich von etwa 315 nm bis etwa 380 nm aufweisen.

Gemäß verschiedenen Ausführungsformen kann das Benetzen von Haar mit einer Fluoreszenzfarbstofflösung das Benetzen der Haarprobe für eine vorbestimmte Zeitspanne aufweisen.

Gemäß verschiedenen Ausführungsformen kann das Verfahren ferner aufweisen: vor dem Registrieren des Licht, Erzeugen von definierten Bedingung für das Belichten der Haarprobe mit UV-Licht und für das Registrieren des Lichts.

Gemäß verschiedenen Ausführungsformen können für das Ermitteln des Schädigungsgrads des Haars Ergebnisse von Vergleichsmessungen genutzt werden.
In verschiedenen Ausführungsbeispielen wird eine Vorrichtung zum Ermitteln eines Schädigungsgrads von Haar bereitgestellt. Die Vorrichtung kann eine UV-Lampe zum Belichten einer Haarprobe mit UV-Licht, eine Vorrichtung zum Registrieren von Fluoreszenzlicht, welches der Haarprobe emittiert wird, und eine Datenverarbeitungsvorrichtung zum Ermitteln einer Fluoreszenzintensität anhand des registrierten Lichts und zum Ermitteln des Schädigungsgrads des Haars unter Einbeziehung der Fluoreszenzintensität aufweisen. Das Fluoreszenzlicht kann eine Eigenfluoreszenz des geschädigten Haars sein und/oder eine Fluoreszenz von in der Haarprobe adsorbiertem Fluoreszenzfarbstoff. Die Haarprobe kann dem Haar entnommen sein, beispielsweise zum Benetzen des Haars mit einer Fluoreszenzfarbstofflösung und/oder für das Registrieren des Fluoreszenzlichts, oder die Haarprobe kann an einem Kopf verbleiben, beispielsweise bei einem Registrieren der Eigenfluoreszenz des Haars.
In verschiedenen Ausführungsbeispielen wird ein Verfahren zum Ermitteln eines nutzerspezifischen Mittels zur Haarbehandlung bereitgestellt. Das Verfahren kann ein Ermitteln eines Schädigungsgrads von Haar eines Nutzers gemäß verschiedenen Ausführungsbeispielen, ein computergestütztes Ermitteln von mittels einer Mehrzahl von Mitteln zur Haarbehandlung erzielbaren Behandlungsergebnissen mittels prädiktiver Analytik unter Einbeziehung des ermittelten Schädigungsgrads, und ein Auswählen des nutzerspezifischen Mittels anhand der ermittelten Behandlungsergebnisse aufweisen.
Gemäß verschiedenen Ausführungsformen kann das nutzerspezifische Mittel zur Haarbehandlung ein Haarpflegeprodukt, ein Haarfärbeprodukt, ein Blondierungsprodukt oder ein Stylingprodukt (z.B. für eine Haarglättung oder eine Dauerwelle) aufweisen. Ein Verfahren gemäss der Erfindung ist in Anspruch 1 definiert. Eine Vorrichtung gemäss der Erfindung ist in Anspruch 12 definiert

Ausführungsbeispiele der Erfindung sind in den Figuren dargestellt und werden im Folgenden näher erläutert.
Es zeigen
Figur 1A und 1B eine schematische Darstellung eines Verfahrens zum Ermitteln eines Schädigungsgrads von Haar gemäß verschiedenen Ausführungsbeispielen, wobei in FIG. 1B eine Vorrichtung zum Ermitteln eines Schädigungsgrads von Haar gemäß verschiedenen Ausführungsbeispielen genutzt wird;
Figur 2 Aufnahmen von Fluoreszenz verschiedener Fluoreszenzfarbstoffe (und ggf. Eigenfluoreszenz) von Haarproben mit unterschiedlichen Schädigungsgraden gemäß verschiedenen Ausführungsbeispielen;
Figur 3 eine schematische Darstellung einer Verwendung von ermittelten Fluoreszenzintensitäten in einem Verfahren zum Ermitteln eines Schädigungsgrads von Haar gemäß verschiedenen Ausführungsbeispielen;
Figur 4 ein Ablaufdiagramm, welches ein Verfahren zum Ermitteln eines Schädigungsgrads von Haar gemäß verschiedenen Ausführungsbeispielen darstellt;
Figur 5 ein Ablaufdiagramm, welches ein Verfahren zum Ermitteln eines Schädigungsgrads von Haar gemäß verschiedenen Ausführungsbeispielen darstellt; und
Figur 6 ein Ablaufdiagramm, welches ein Verfahren zum Ermitteln eines nutzerspezifischen Mittels zur Haarbehandlung gemäß verschiedenen Ausführungsbeispielen darstellt.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

In der vorliegenden Beschreibung werden die Begriffe Prädiktive Analytik, Predictive Analytics, Big Data und Data Mining synonym verwendet.

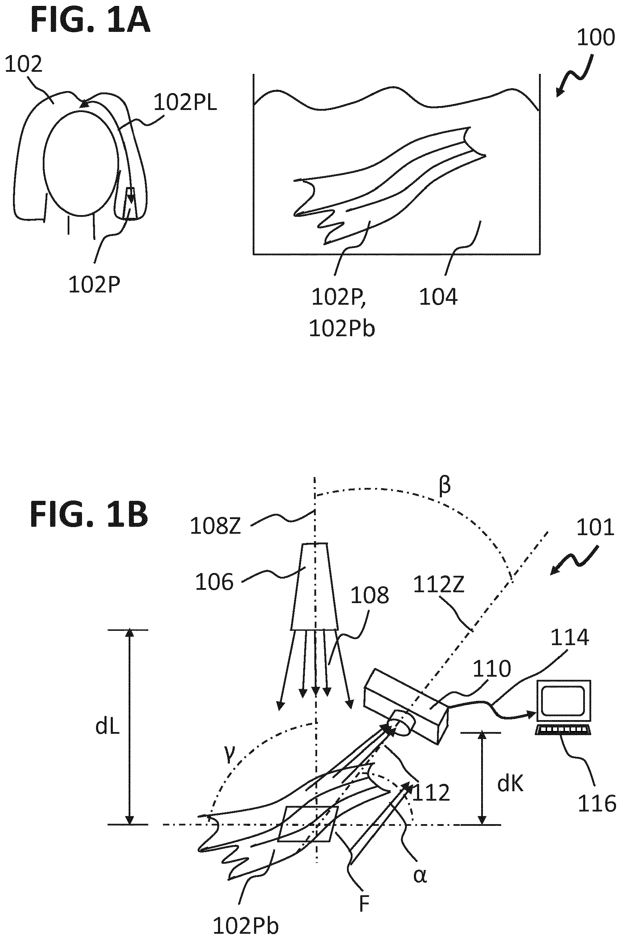

Figur 1A und 1B zeigen in Ansichten 100, 101 eine schematische Darstellung eines Verfahrens zum Ermitteln eines Schädigungsgrads von Haar gemäß verschiedenen Ausführungsbeispielen.

Gemäß verschiedenen Ausführungsbeispielen kann zum Ermitteln eines Schädigungsgrads von Haar 102 eines Nutzers eine Haarprobe 102P entnommen werden. Die Haarprobe 102P kann beispielsweise in einer Entfernung 102PL von einer Kopfhaut des Nutzers entnommen werden. Die Haarprobe 102P kann eine Mindestmenge von Haaren aufweisen, die beispielsweise ausgedrückt sein kann als eine minimale Fläche, welche flächendeckend mit der (z.B. flach ausgebreiteten) Haarprobe 102P abgedeckt sein kann, beispielsweise mindestens 1 cm², oder beispielsweise als ein Mindestgewicht, beispielsweise mindestens 0,5 g.

Gemäß verschiedenen Ausführungsbeispielen kann ein Ermitteln des Schädigungsgrads von Haar 102 eines Nutzers auch ohne ein Entnehmen einer Haarprobe 102P erfolgen. Das Registrieren von Fluoreszenzlicht 112 der Haarprobe 102P kann beispielsweise am Kopf des Nutzers erfolgen, beispielsweise bei einem Registrieren von Eigenfluoreszenz des geschädigten Haars 102, und/oder in einem Fall, in welchem ein Benetzen der Haarprobe 102P mit einer Fluoreszenzfarbstofflösung 104 am Kopf möglich ist.

In verschiedenen Ausführungsbeispielen kann die Haarprobe 102P mit einer Fluoreszenzfarbstofflösung 104 benetzt werden, beispielsweise mittels Eintauchens in die Fluoreszenzfarbstofflösung 104. Die Fluoreszenzfarbstofflösung 104 kann in einem Lösungsmittel, beispielsweise Wasser, gelösten Fluoreszenzfarbstoff aufweisen, beispielsweise Rhodamin B, Coumarin und/oder Fluorescein, und/oder einen anderen Fluoreszenzfarbstoff, der ähnlich wie die genannten Fluoreszenzfarbstoffe die Eigenschaft hat, von geschädigtem Haar stärker adsorbiert zu werden. Der Fluoreszenzfarbstoff hat die bekannte Eigenschaft, beim Belichten mit UV-Licht 108 angeregt zu werden und infolge der Anregung längerwelliges Licht 112 abzustrahlen. Das abgestrahlte Licht 112 wird auch als Fluoreszenzlicht 112 bezeichnet.

Die Fluoreszenzfarbstofflösung 104 kann hochverdünnt sein. Eine Konzentration des Fluoreszenzfarbstoffs in der Fluoreszenzfarbstofflösung 104 kann in verschiedenen Ausführungsbeispielen in einem Bereich von etwa 0,01% (Gew.) bis etwa 0,1% (Gew.), beispielsweise von etwa 0,01% bis etwa 0,07% sein. Eine Konzentration von Rhodamin B in der Fluoreszenzfarbstofflösung 104 kann beispielsweise etwa 0,05% (Gew.) betragen. Eine Konzentration von Fluorescein in der Fluoreszenzfarbstofflösung 104 kann beispielsweise etwa 0,04% (Gew.) betragen. Eine Konzentration von Coumarin in der Fluoreszenzfarbstofflösung 104 kann beispielsweise etwa 0,02% (Gew.) betragen.

Das Benetzen der Haarprobe 102P mit der Fluoreszenzfarbstofflösung 104 kann in verschiedenen Ausführungsbeispielen für eine vorbestimmte Zeitspanne erfolgen, beispielsweise eine Sekunde, zehn Sekunden, eine Minute, fünf Minuten, zehn Minuten oder jede andere geeignete vorbestimmte Zeitspanne, oder beispielsweise für eine Mindestdauer, beispielsweise mindestens eine Sekunde, zehn Sekunden, eine Minute, fünf Minuten, zehn Minuten oder jede andere geeignete vorbestimmte Mindestdauer. Während des Benetzens der Haarprobe 102P mit der Fluoreszenzfarbstofflösung 104 kann der Fluoreszenzfarbstoff von der Haarprobe 102P, insbesondere von geschädigten Haaren in der Haarprobe 102P, zumindest teilweise adsorbiert werden. Die benetzte Haarprobe 102P wird als 102Pb bezeichnet.

In verschiedenen Ausführungsbeispielen kann im Anschluss an das Benetzen der Haarprobe 102P überschüssige, d.h nicht von der Haarprobe 102Pb adsorbierte Fluoreszenzfarbstofflösung 104, entfernt werden. Die Haarprobe 102Pb kann beispielsweise gespült werden, beispielsweise mit Wasser oder, falls die Fluoreszenzfarbstofflösung 104 ein anderes Lösungsmittel als Wasser enthält, beispielsweise mit diesem Lösungsmittel. Allgemein kann jedes Verfahren verwendet werden, welches geeignet ist, die überschüssige Fluoreszenzfarbstofflösung 104 zu entfernen und den adsorbierten Fluoreszenzfarbstoff in der Haarprobe 102P zu belassen, ohne die Haarprobe 102Pb weiter zu schädigen oder zu verändern. Durch das Entfernen der überschüssigen Fluoreszenzfarbstofflösung 104 kann eine fehlerhafte Messung einer Fluoreszenzintensität der Haarprobe 102Pb vermieden werden.

Nach dem Entfernen der überschüssigen Fluoreszenzfarbstofflösung 104 kann die Fluoreszenzintensität der Haarprobe 102Pb ermittelt werden.

In verschiedenen Ausführungsbeispielen kann dazu eine Vorrichtung zum Ermitteln des Schädigungsgrads des Haars 102 gemäß verschiedenen Ausführungsbeispielen genutzt werden, wie sie in FIG. 1B beispielhaft schematisch in einer Ansicht 101 dargestellt ist.

In verschiedenen Ausführungsbeispielen kann die Haarprobe 102Pb flächendeckend ausgebreitet werden.

In verschiedenen Ausführungsbeispielen kann die Haarprobe 102Pb mit UV-Licht 108 beleuchtet werden. Das UV-Licht kann eine Wellenlänge in einem Bereich von etwa 315 nm bis etwa 380 nm aufweisen, beispielsweise in einem Bereich von etwa 350 nm bis etwa 375 nm, beispielsweise ungefähr 370 nm. In verschiedenen Ausführungsbeispielen kann UV-Licht mit einer Wellenlänge von weniger als 315 nm genutzt werden.

In verschiedenen Ausführungsbeispielen kann eine Lichtquelle 106 des UV-Lichts 108 beispielsweise eine UV-Lampe oder eine UV-LED sein. Allgemein kann jedoch eine beliebige Lichtquelle genutzt werden, die UV-Licht 108 im genannten Wellenlängenbereich in einer Intensität erzeugt, die für eine standardisierte Messung einer Fluoreszenzintensität wie unten beschrieben ausreichend ist. Beispielsweise kann als Lichtquelle 106 eine herkömmliche UV-Lampe genutzt werden, wie sie beispielsweise bei einer Echtheitsprüfung von Geldscheinen verwendet wird.

In verschiedenen Ausführungsbeispielen kann die Lichtquelle 106 in einem Abstand dL von der Haarprobe 102Pb angeordnet sein, und die Haarprobe unter einem Winkel γ beleuchten, wobei der Winkel γ gemessen sein kann zwischen einer Ebene, in der die Haarprobe 102Pb flächig ausgebreitet ist, und einem Zentralstrahl 108Z des UV-Lichts, welcher in verschiedenen Ausführungsbeispielen mit einer optischen Achse der Lichtquelle 106 zusammenfallen kann.

In verschiedenen Ausführungsbeispielen kann die Lichtquelle 106 eine vorbestimmte Abstrahl-Lichtstärke aufweisen.

In verschiedenen Ausführungsbeispielen können die Abstrahl-Lichtstärke, der Winkel γ und der Abstand dL so eingerichtet sein, dass ein Bereich F der Haarprobe 102Pb mit einer vorbestimmten Belichtungsintensität, beispielsweise einer vorbestimmten Mindestbelichtungsintensität, belichtet wird.

In verschiedenen Ausführungsbeispielen kann während eines Belichtens der Haarprobe 102Pb mit dem UV-Licht 108 Licht 112 (Fluoreszenzlicht 112) registriert werden, welches von in der Haarprobe 102Pb adsorbiertem Fluoreszenzfarbstoff emittiert wird.

In verschiedenen Ausführungsbeispielen kann zum Registrieren des Lichts 112 eine Vorrichtung 110 zum Registrieren des Licht 112, welches von in der Haarprobe adsorbiertem Fluoreszenzfarbstoff emittiert wird, genutzt werden.

Die Vorrichtung 110 zum Registrieren des Licht 112 kann in einem Abstand dK von der Haarprobe 102Pb angeordnet sein, und das Licht der Haarprobe 102Pb unter einem Winkel α registrieren, wobei der Winkel α gemessen sein kann zwischen einer Ebene, in der die Haarprobe 102Pb flächig ausgebreitet ist, und einem Zentralstrahl 112Z des registrierten Lichts 112, welcher in verschiedenen Ausführungsbeispielen mit einer optischen Achse der Vorrichtung 110 zum Registrieren des Lichts 112 zusammenfallen kann.

Die Vorrichtung 110 zum Registrieren des Lichts 112 und die Lichtquelle 106 können zueinander in einem Winkel β angeordnet sein. Der Winkel β kann zwischen dem Zentralstrahl 112Z des registrierten Lichts 112 und dem Zentralstrahl 108Z des UV-Lichts gemessen werden.

Die Vorrichtung 110 zum Registrieren des Lichts 112 kann beispielsweise eine Kamera, z.B. eine Digitalkamera, aufweisen. Das Registrieren kann ein Erzeugen eines Fotos, beispielsweise eines Digitalfotos aufweisen. Die Digitalkamera kann beispielsweise Teil eines Smartphones, eines Tablets oder eines Laptops, d.h. einer mobilen Datenverarbeitungsvorrichtung sein. Allgemein kann allerdings jede Vorrichtung 110 zum Registrieren des Lichts 112 genutzt werden, die eine Zuordnung von empfangener Intensität und registrierter Information über die empfangene Intensität erlaubt, beispielsweise eine analoge Kamera, deren aufgenommenes Bild anschließend quantitativ hinsichtlich der registrierten Intensität weiterverarbeitet werden kann.

In verschiedenen Ausführungsbeispielen können der Abstand dK, der Winkel α so eingerichtet sein, dass Licht einer vorbestimmten Fläche, beispielsweise der Fläche F, von der Vorrichtung 110 zum Registrieren des Lichts 112 registriert wird. Beispielsweise können die genannten Parameter so abgestimmt sein, dass die vorbestimmte Fläche eine vorbestimmte Größe, z.B. eine Mindest- oder eine Maximalgröße, aufweist, beispielsweise eine Fläche von mindestens 1 cm², beispielsweise eine Fläche von maximal 10 cm².

Gemäß verschiedenen Ausführungsbeispielen können die Abstände dL und dK und die Winkel α, β und γ (und nicht dargestellte räumliche Positionen der Lichtquelle 106, der Haarprobe 106Pb und der Vorrichtung 110 zum Registrieren des Lichts 112) so abgestimmt sein, dass bei einer vorgegebenen Haarprobe 102Pb möglichst viel Licht 112 in der Vorrichtung 110 zum Registrieren des Lichts 112 registriert werden kann.

In verschiedenen Ausführungsbeispielen können die genannten Parameter so abgestimmt sein, dass sie sich von einem Nutzer, beispielsweise im privaten Bereich oder im Dienstleistungsbereich, leicht verwirklichen lassen.

In verschiedenen Ausführungsbeispielen können die genannten Parameter so abgestimmt sein, dass sie den Parametern entsprechen, welche bei Referenzmessungen, welche an standardisierten Haarproben ausgeführt werden, genutzt werden.

Für eine weitergehende Standardisierung kann in verschiedenen Ausführungsbeispielen (nicht dargestellt) gleichzeitig mit der Haarprobe 102Pb oder nacheinander Licht einer Referenzprobe, beispielsweise eines mit Fluoreszenzfarbstofflösung getränkten Materials mit bekannter Adsorptionsfähigkeit für den Fluoreszenzfarbstoff, von der Vorrichtung 110 zum Registrieren des Lichts 112 registriert werden.

In verschiedenen Ausführungsbeispielen kann das Verfahren zum Ermitteln eines Schädigungsgrads von Haar ferner ein Ermitteln einer Fluoreszenzintensität anhand des registrierten Lichts 112 aufweisen.

In verschiedenen Ausführungsbeispielen kann dazu das registrierte Licht 112 in verarbeiteter Form, beispielsweise als Digitalfoto oder eine andere Quantifizierung des registrierten Lichts, an eine Datenverarbeitungsvorrichtung 116 übertragen werden. Dies ist mit dem Bezugszeichen 114 gekennzeichnet. Die Übertragung kann auf bekanntem Weg erfolgen, beispielsweise mittels eines Datenkabels, kabelloser Datenübertragung (z.B. Wlan, ZigBee, Thread, Bluetooth oder Near Field Communication (NFC)), oder eine Übertragung kann innerhalb einer Vorrichtung erfolgen, wenn die Vorrichtung 110 zum Registrieren des Lichts 112, wie oben beschrieben, Teil einer Datenverarbeitungsvorrichtung (z.B. Mobiltelefon, Tablet, Laptop) ist, welche auch eine Funktion der Datenverarbeitungsvorrichtung 116 erfüllen kann.

Zum Empfangen und Weiterverarbeiten der Daten kann die Datenverarbeitungsvorrichtung beispielsweise mit einer entsprechenden Software ausgestattet sein, beispielsweise einer App.

In verschiedenen Ausführungsbeispielen kann zunächst mittels bildanalytischer Verfahren anhand des registrierten Lichts 112, welches beispielsweise unter normierten Bedingungen registriert wurde, beispielsweise mittels Vorbestimmens unter anderem der oben genannten Parameter, eine Maßzahl für das registrierte Licht 112 ermittelt werden, beispielsweise eine Zahl von Counts (Zählwerten) in einem vorbestimmten Bildbereich oder ähnliches.

In verschiedenen Ausführungsbeispielen kann anhand der ermittelten Maßzahl für das registrierte Licht 112 eine Fluoreszenzintensität ermittelt werden. Hierfür können
mathematische Modelle aus dem Bereich Prädiktive Analytik genutzt werden, um eine Beziehung zwischen der Maßzahl für das registrierte Licht 112 und einer zugehörigen standardisierten Fluoreszenzintensität, beispielsweise unter Berücksichtigung weiterer Parameter wie z.B. einer Leuchtstärke der Lichtquelle 106, der oben genannten Abstände und Winkel, einer Apertur der Vorrichtung 110 zum Registrieren des Lichts 112, und weiterer Einflussgrößen, welche ein Verhältnis von Fluoreszenzintensität und registriertem Licht beeinflussen können.
In verschiedenen Ausführungsbeispielen kann das von der Referenzprobe registrierte Licht in die Ermittlung der Maßzahl einbezogen werden.

In verschiedenen Ausführungsbeispielen kann anhand der ermittelten Fluoreszenzintensität ein Cysteinsäuregehalt ermittelt werden. Hierfür können wiederum mathematische Modelle aus dem Bereich Prädiktive Analytik genutzt werden, diesmal um eine Beziehung zwischen der (standardisierten) Fluoreszenzintensität und einem zugehörigen Cysteinsäuregehalt (und damit einem Schädigungsgrad der Haarprobe 102Pb) zu ermitteln.

Als unabhängige Parameter können in das Modell dabei in verschiedenen Ausführungsbeispielen beispielsweise eine Beziehung zwischen Cysteinsäuregehalt und Fluoreszenzintensität bzw. entsprechende Datenwerte, z.B. in Form zugeordneter Datenpaare, einbezogen werden, welche mittels Vermessung von Standard-Haarproben, welche einen anhand bekannter aufwändiger Verfahren ermittelten Cysteinsäuregehalt aufweisen ermittelt bzw. mathematisch modelliert wurden.

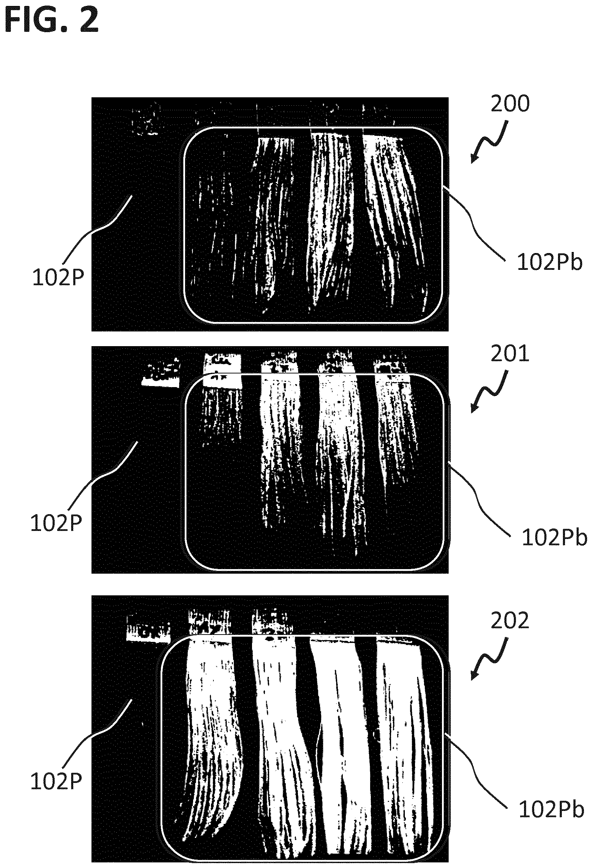

Figur 2 zeigt Aufnahmen 200, 201, 202 von Fluoreszenz verschiedener Fluoreszenzfarbstoffe von Standard-Haarproben mit unterschiedlichen Schädigungsgraden gemäß verschiedenen Ausführungsbeispielen.

Hierbei sind jeweils die linken Haarproben 102P unbehandelt (wurde 0x ultrablondiert), die jeweils rechts davon angeordneten Haarproben 102Pb sind mit einer Fluoreszenzfarbstofflösung benetzt worden. Dabei wurden (von oben nach unten) die Fluoreszenzfarbstoffe Rhodamin B, Cumarin bzw. Fluorescein verwendet.

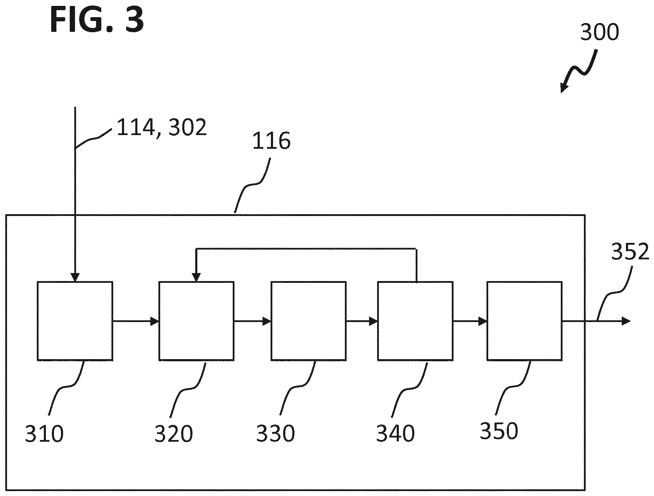

Die dargestellten benetzten Haarproben 102Pb wurden verschieden stark geschädigt, und zwar (jeweils von links nach rechts) durch 1-, 2, 3, bzw. 4-maliges Ultrablondieren.
Eine jeweils von links nach rechts zunehmende Fluoreszenzintensität ist anhand der Fotos ersichtlich. Diese und weitere oder ähnliche Standardproben können zum Erstellen der Beziehung zwischen Cysteinsäuregehalt und Fluoreszenzintensität bzw. entsprechende Datenwerte, welche zum Ermitteln des Schädigungsgrads einer neuen Haarprobe 102P verwendet werden (diese und ggf. weitere Daten sind in FIG. 3 mit 302 gekennzeichnet), genutzt werden.

Figur 3 eine schematische Darstellung 300 einer Verwendung von ermittelten Fluoreszenzintensitäten in einem Verfahren zum Ermitteln eines Schädigungsgrads von Haar gemäß verschiedenen Ausführungsbeispielen.

In verschiedenen Ausführungsbeispielen kann mittels der Prädiktiven Analytik ein kontinuierliches Modell der Fluoreszenzintensität und des Schädigungsgrads erzeugt werden, so dass es möglich ist, für einen Wert einer Fluoreszenzintensität oder einer Kombination der Fluoreszenzintensität mit weiteren, eine Zuordnung von Fluoreszenzintensität und Schädigungsgrad beeinflussenden Parametern auch dann mit Hilfe des Modells einen Wert für den Schädigungsgrad zu ermitteln, wenn die eingegebene Kombination von Daten keinem der entsprechenden experimentellen Werte bzw. Wertekombinationen entspricht.

In verschiedenen Ausführungsbeispielen kann der Schädigungsgrad in einer kategorialen Skala (z.B. leicht, mittel, schwer) ermittelt werden.

In verschiedenen Ausführungsbeispielen kann der Schädigungsgrad in einer metrischen Skala (z.B. Prozentanteil des Gehalts an Cysteinsäure) ermittelt werden.

In verschiedenen Ausführungsbeispielen können die weiteren Parameter, die beispielsweise eine unterschiedliche Adsorption von Fluoreszenzfarbstoff bei gleichem Schädigungsgrad bewirken können, z.B. eine Haarfarbe (z.B. blond, braun, schwarz, usw.) und/oder eine ethnische Zugehörigkeit des Haartyps (z.B. kaukasisch, asiatisch, afro-amerikanisch) aufweisen.

Prädiktive Analytik kann allgemein als ein Verfahren beschrieben werden, um aus großen Datenmengen Informationen zu extrahieren und aus diesen Daten ein Modell zu erzeugen, welches es erlaubt, auch für Werte, die nicht Teil des Datensatzes sind, Vorhersagen zu treffen. Bei Anwendung eines Prädiktive Analytik Verfahrens kann typischerweise ein Teil des Datensatzes als Trainings-Datensatz (auch als Trainingssatz oder Trainingsdaten bezeichnet) genutzt werden. Anhand dieses Trainingsdatensatzes können ein oder mehrere Modelle erzeugt werden, welche dann anhand der Daten, die nicht Teil des Trainingsdatensatzes sind, anhand der gesamten Daten, oder anhand eines speziell ausgewählten Teils der Daten, getestet werden können.

Für eine Bewertung des Modells, also eine Ermittlung der Anpassungsgüte, können beispielsweise ein Bestimmtheitsmaß R², ein mittlerer absoluter Fehler, ein mittlerer quadratischer Fehler, eine Standardabweichung und/oder eine mittlere Abweichung herangezogen werden.

Das Bestimmtheitsmaß R² kann für ein lineares Regressionsmodell einem quadrierten Korrelationskoeffizienten entsprechen. Für ein anderes Modell (eine andere Beziehung) kann es anders definiert sein.

Für die Modellierung mittels Prädiktiver Analytik können gemäß verschiedenen Ausführungsbeispielen verschiedene Funktionen oder Verfahren herangezogen werden. In einem einfachen Fall kann beispielsweise eine multiple lineare Regression genutzt werden. Bessere Ergebnisse können typischerweise unter Verwendung von polynomen Regressionen, neuronalen Netzen, Support Vector Machines, Entscheidungsbäumen (beispielsweise Baum-Ensembles) oder ähnlichem erzielt werden.

In verschiedenen Ausführungsbeispielen kann das beschriebene Verfahren zur computergestützten Vorhersage von Eigenschaften von Haarfarben mittels einer Datenverarbeitungsvorrichtung 116 ausgeführt werden.

Die Datenverarbeitungsvorrichtung kann, wie oben im Zusammenhang mit FIG. 1B beschrieben, beispielsweise eine mobile Datenverarbeitungsvorrichtung, beispielsweise ein Smartphone, ein Tablet oder einen Laptop, aber auch einen sonstigen Computer aufweisen, oder jede andere Datenverarbeitungsvorrichtung, die geeignet ist, die Daten zu speichern und bereitzustellen und das Prädiktive Analytik Verfahren auszuführen, also beispielsweise jede Datenverarbeitungsvorrichtung, beispielsweise ein Smart Mirror, mit hinreichend großem Datenspeicher und hinreichend leistungsfähigem Prozessor.

In verschiedenen Ausführungsbeispielen kann die Datenverarbeitungsvorrichtung mindestens eine Eingabevorrichtung zum Eingeben von Informationen in die Datenverarbeitungsvorrichtung aufweisen, beispielsweise zum Eingeben der Haarfarben-Daten und ggf. zum Eingeben von Anweisungen, Parametern usw. für ein Ausführen des Verfahrens. Die Eingabevorrichtung kann beispielsweise eine Tastatur, einen berührungsempfindlichen Bildschirm oder ein Mikrophon umfassen.

In verschiedenen Ausführungsbeispielen kann die Datenverarbeitungsvorrichtung mindestens eine Ausgabevorrichtung zum Ausgeben von Informationen aufweisen, beispielsweise zum Ausgeben von Ergebnissen des Verfahrens.

In verschiedenen Ausführungsbeispielen kann die mindestens eine Ausgabevorrichtung einen Bildschirm und/oder einen Drucker und/oder Lautsprecher aufweisen.

In verschiedenen Ausführungsbeispielen kann für eine Modellierung mittels prädiktiver Analytikjedes beliebige Programm (z.B. eine App) genutzt werden, welches eine solche Funktionalität bereitstellt.

Die in Figur 3 dargestellten Komponenten 310, 320, 330, 340, 350 können einzelne während der Ausführung des Prädiktive-Analytik-Verfahrens durchgeführte Prozesse darstellen.

In verschiedenen Ausführungsbeispielen können bei 310 die ermittelten Fluoreszenzintensitätswerte 114 und weitere Parameter 302, welche beispielsweise die Datenpaare der Referenzmessungen aufweisen, und ggf. weitere Parameter, vom Prädiktive-Analytik-Programm (z.B. Software, z.B. App) eingelesen werden.

In verschiedenen Ausführungsbeispielen können bei 320 Filter bereitgestellt sein, welche ggf. aus einer Gesamtheit der bei 310 bereitgestellten Daten diejenigen auswählt, die für die Ermittlung des Schädigungsgrads genutzt werden sollen.

In 330 kann gemäß verschiedenen Ausführungsbeispielen eine funktionelle Komponente zum Erstellen eines Modells, z.B. zum Ausführen einer einfachen linearen Regression für die bei 320 ausgewählten Daten, bereitgestellt sein.

Mittels des Modells, z.B. der Regression, kann eine Beziehung zwischen der Mehrzahl von Eingangsparametern, welche die Fluoreszenzintensität und ggf. weitere Parameter aufweisen, und dem Schädigungsgrad ermittelt werden. Diese gefundene Beziehung kann auch als Modell bezeichnet werden. Ein Prinzip für eine Anpassung des Modells an die Daten kann eine Optimierung des kleinsten-Quadrate-Fehlers sein. Anders ausgedrückt kann ein Fehler mittels der Methode der kleinsten Quadrate minimiert werden.

In verschiedenen Ausführungsbeispielen kann bei 340 ein Vorhersager bereitgestellt sein, welchem sowohl die Ergebnisse des Modells, z.B. der einfachen linearen Regression, als auch ggf. ungenutzte (nicht ausgewählte) Haarfarben-Daten aus 320 zugeführt werden können. Der Vorhersager kann für sämtliche ausgewählten Färbevoraussetzungsparameter unter Anwendung der zuvor bestimmten Beziehung Werte für den Schädigungsgrad bestimmen.

In verschiedenen Ausführungsbeispielen kann bei 350 ein Bewerter bereitgestellt sein, welcher unter Verwendung der mittels des Vorhersagers bestimmten Werte für den modellierten Schädigungsgrad und den tatsächlich gemessenen Schädigungsgrad (für die Referenzdaten 203) Werte berechnet, welche als Maß für eine Anpassungsgüte des Modells genutzt werden können. Beispielsweise können ein Bestimmtheitsmaß R² (welches für eine lineare Regression einem quadrierten Korrelationskoeffizienten entspricht; generell können gute Werte für das Bestimmtheitsmaß R² etwa zwischen 0,8 und 1,0 liegen), ein mittlerer absoluter Fehler, ein mittlerer quadratischer Fehler, eine Standardabweichung und/oder eine mittlere absolute Abweichung bestimmt werden. Besonderes Augenmerk kann in verschiedenen Ausführungsbeispielen auf das Bestimmtheitsmaß R² und auf die mittlere absolute Abweichung gelegt werden.

In verschiedenen Ausführungsbeispielen kann bei 352 das Ergebnis der Ermittlung des Schädigungsgrads des Haars ausgegeben, z.B. angezeigt, werden.

In verschiedenen Ausführungsbeispielen kann bei der Ermittlung des Schädigungsgrads des Haars 102 ferner die Entfernung 102PL (siehe FIG. 1A) einbezogen werden, welche die Haarprobe 102P von der Kopfhaut des Nutzers hatte. Typischerweise, zumindest bei längerem Haar, ist das Haar 102 an den Haarspitzen stärker geschädigt als nahe der Kopfhaut. Dementsprechend kann anhand der Entfernung 102PL und einer Gesamtlänge des Haars 102 beispielsweise mittels des Modells eine mittlere Schädigung des Haars oder z.B. eine maximale Schädigung des Haars ermittelt werden.

In verschiedenen Ausführungsbeispielen können anstelle des Prädiktive-Analytik-Verfahrens andere, z.B. einfachere, Verfahren zur Zuordnung des Schädigungsgrads zur bestimmten Fluoreszenzintensität genutzt werden.

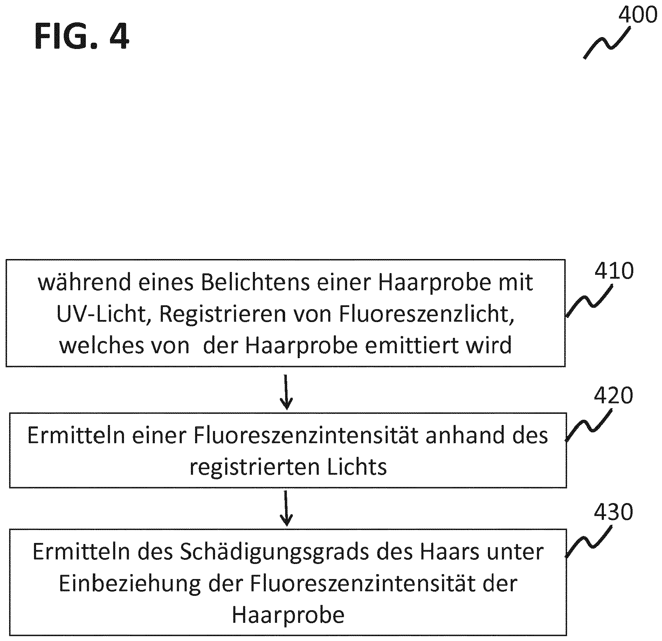

Figur 4 zeigt ein Ablaufdiagramm 400, welches ein Verfahren zum Ermitteln eines Schädigungsgrads von Haar gemäß verschiedenen Ausführungsbeispielen darstellt.

In verschiedenen Ausführungsbeispielen kann das Verfahren aufweisen während eines Belichtens einer Haarprobe mit UV-Licht, Registrieren von Fluoreszenzlicht, welches von der Haarprobe emittiert wird (bei 410), Ermitteln einer Fluoreszenzintensität anhand des registrierten Lichts (bei 420) und Ermitteln des Schädigungsgrads des Haars unter Einbeziehung der Fluoreszenzintensität der Haarprobe (bei 430). Das Fluoreszenzlicht kann beispielsweise eine Eigenfluoreszenz des geschädigten Haars sein.

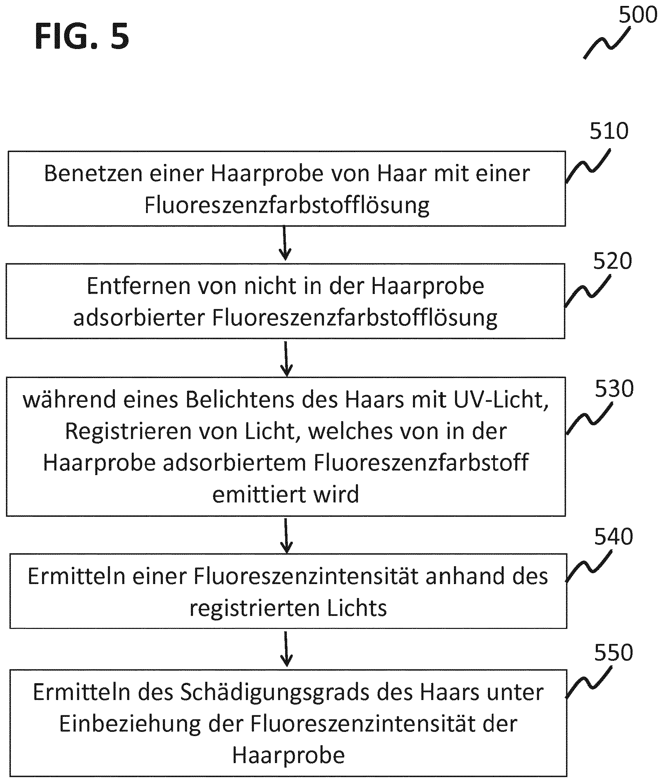

Figur 5 zeigt ein Ablaufdiagramm 500, welches ein Verfahren zum Ermitteln eines Schädigungsgrads von Haar gemäß verschiedenen Ausführungsbeispielen darstellt.

In verschiedenen Ausführungsbeispielen kann das Verfahren aufweisen ein Benetzen einer dem Haar entnommenen Haarprobe mit einer Fluoreszenzfarbstofflösung (bei 510), ein Entfernen von nicht in der Haarprobe adsorbierter Fluoreszenzfarbstofflösung (bei 520), während eines Belichtens der Haarprobe mit UV-Licht, Registrieren von Licht, welches von in der Haarprobe adsorbiertem Fluoreszenzfarbstoff emittiert wird (bei 530), Ermitteln einer Fluoreszenzintensität anhand des registrierten Lichts (bei 540) und Ermitteln des Schädigungsgrads des Haars unter Einbeziehung der Fluoreszenzintensität der Haarprobe (bei 550).

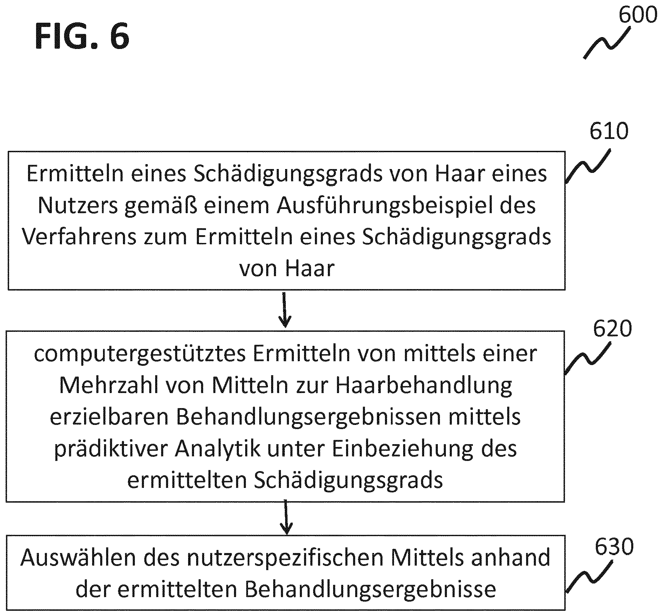

Figur 6 zeigt ein Ablaufdiagramm 600, welches ein Verfahren zum Ermitteln eines nutzerspezifischen Mittels zur Haarbehandlung gemäß verschiedenen Ausführungsbeispielen darstellt.

In verschiedenen Ausführungsbeispielen kann das oben beschriebene Verfahren zum Ermitteln eines Schädigungsgrads von Haar bzw. die oben beschriebene Vorrichtung zum Ermitteln eines Schädigungsgrads von Haar genutzt werden für ein Verfahren zum Ermitteln eines nutzerspezifischen Mittels zur Haarbehandlung.

In verschiedenen Ausführungsbeispielen kann das Verfahren zum Ermitteln eines nutzerspezifischen Mittels zur Haarbehandlung aufweisen: ein Ermitteln eines Schädigungsgrads von Haar eines Nutzers gemäß einer der oben beschriebenen Ausführungsbeispiele (bei 610), ein computergestütztes Ermitteln von mittels einer Mehrzahl von Mitteln zur Haarbehandlung erzielbaren Behandlungsergebnissen mittels prädiktiver Analytik unter Einbeziehung des ermittelten Schädigungsgrads (bei 620), und ein Auswählen des nutzerspezifischen Mittels anhand der ermittelten Behandlungsergebnisse (bei 630).

In verschiedenen Ausführungsbeispielen kann das nutzerspezifische Mittel zur Haarbehandlung ein Haarpflegeprodukt, ein Haarfärbeprodukt, ein Blondierungsprodukt, oder ein Stylingprodukt, beispielsweise ein Glättungsmittel wie z.B. Straightener oder Relaxer oder ein Mittel für eine Dauerwelle, aufweisen.

In verschiedenen Ausführungsbeispielen kann das Verfahren zum Ermitteln eines nutzerspezifischen Mittels zur Haarbehandlung ferner die Einleitung eines Bestellvorgangs, vorzugsweise durch Aufrufen einer Internetseite eines Herstellers von individuellen Haarpflegeprodukten, individuellen Haarfärbeprodukten, individuellen Blondierungsprodukten oder ein individuellen Stylingprodukten, aufweisen.

Immer Kunden wünschen sich ein individuell auf ihre Bedürfnisse abgestimmtes Produkt. Dabei kann es sich um ein speziell für den Kunden hergestelltes Produkt oder ein sogenanntes "mass customized" Produkt handeln. Bei einem "mass customized" Produkt kann durch Variation von wenigen, aus Kundensicht jedoch entscheidenden Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurator abläuft. Dieser Konfigurator hilft dem Kunden bei der Auswahl der Merkmale/Inhaltsstoffe und weist ihn auf die zulässigen/unzulässigen Merkmalskombinationen hin, wobei letztere dann nicht ausgewählt werden können.

Bei Haarpflegeprodukten, Haarfärbeprodukten, Blondierungsprodukten oder Stylingprodukten umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten Schädigungsgrad ungeeigneter Inhaltsstoffe vermieden werden. Umgekehrt kann die Auswahl für den ermittelten Schädigungsgrad geeigneter Inhaltsstoffe durch den Produktkonfigurator vorgegeben oder vorgeschlagen werden.

In verschiedenen Ausführungsbeispielen kann bei bestimmten Haarschädigungsgraden der Besuch eines Friseurs empfohlen werden. In verschiedenen Ausführungsbeispielen kann direkt über die Software/App, welche den Schädigungsgrad ermittelt, ein Buchungsvorgang eingeleitet werden. Dazu können beispielsweise in der Software/App die Kontaktdaten von Friseuren hinterlegt sein und diese dem Nutzer angezeigt werden. Zusätzlich kann über Filter, wie beispielsweise die Postleitzahl, die Auswahl eingeschränkt werden. Alternativ kann die Buchung eines Friseurtermins über eine separate Software/App, wie beispielsweise Treatwell, erfolgen.

Weitere vorteilhafte Ausgestaltungen des Verfahrens ergeben sich aus der Beschreibung der Vorrichtung und umgekehrt.

## Patentansprüche

1. Verfahren zum Ermitteln eines Schädigungsgrads von Haar (102), aufweisend:
während eines Belichtens einer Haarprobe (102P) des Haars mit UV-Licht (108),
Registrieren von Licht, welches von der Haarprobe emittiert wird;
Ermitteln einer Fluoreszenzintensität anhand des registrierten Lichts; und
Ermitteln des Schädigungsgrads des Haars unter Einbeziehung der Fluoreszenzintensität der Haarprobe,
wobei das Ermitteln des Schädigungsgrads durch eine Anwendung von einem mathematischen Model erfolgt, wobei das mathematische Model mittels eines Verfahrens aus einer Gruppe von Verfahren erstellt wird, wobei die Gruppe von Verfahren aufweist:
lineare oder multi-lineare Regression,
polynome Regression,
neuronale-Netze-Verfahren,
Support Vector Machine-Verfahren; und
Entscheidungsbäume-Verfahren, wobei das mathematische Model mittels Vermessung von Standard-Haarproben, welche einen ermittelten Cysteinsäuregehalt aufweisen, erstellt wird, welches dann bei der Haarprobe anhand des registrierten Lichts und der daraus ermittelten Fluoreszenzintensität eine Berechnung eines Gehalts an Cysteinsäure, und damit des Schädigungsgrads, durchführt.

2. Verfahren gemäß Anspruch 1, ferner aufweisend:
Benetzen der Haarprobe mit einer Fluoreszenzfarbstofflösung (104); und
Entfernen von nicht in der Haarprobe adsorbierter Fluoreszenzfarbstofflösung;
wobei das registrierte Licht zumindest teilweise von in der Haarprobe adsorbiertem Fluoreszenzfarbstoff emittiert wird.

3. Verfahren gemäß Anspruch 1,
wobei das Ermitteln des Schädigungsgrads aufweist Ermitteln einer Beziehung zwischen einer Mehrzahl von fluoreszenzintensitätbeeinflussenden Parametern und der Fluoreszenzintensität mittels prädiktiver Analytik.

4. Verfahren gemäß Anspruch 3,
wobei die Mehrzahl von fluoreszenzintensitätbeeinflussenden Parametern eine Haarfarbe und/oder eine ethnische Zugehörigkeit eines Typs des Haars aufweist.

5. Verfahren gemäß einem der Ansprüche 1 bis 4,
wobei das Registrieren von Licht ein Erzeugen eines Fotos oder eine direkte Erfassung des Lichts aufweist.

6. Verfahren gemäß einem der Ansprüche 1 bis 5,
wobei das Registrieren von Licht, das
Ermitteln einer Fluoreszenzintensität und/oder das
Ermitteln des Schädigungsgrads des Haars mittels einer mobilen Datenverarbeitungsvorrichtung ausgeführt wird.

7. Verfahren gemäß einem der Ansprüche 2 bis 6,
wobei die Fluoreszenzfarbstofflösung Cumarin aufweist.

8. Verfahren gemäß einem der Ansprüche 1 bis 7,
wobei das UV-Licht eine Wellenlänge in einem Bereich von etwa 315 nm bis etwa 380 nm aufweist.

9. Verfahren gemäß einem der Ansprüche 2 bis 8,
wobei das Benetzen der Haarprobe mit einer Fluoreszenzfarbstofflösung das Benetzen des Haars für eine vorbestimmte Zeitspanne aufweist.

10. Verfahren gemäß einem der Ansprüche 1 bis 9, ferner aufweisend:
vor dem Registrieren des Licht, Erzeugen von definierten Bedingung für das Belichten der Haarprobe mit UV-Licht und für das Registrieren des Lichts.

11. Verfahren gemäß einem der Ansprüche 1 bis 10,
wobei für das Ermitteln des Schädigungsgrads des Haars Ergebnisse von Vergleichsmessungen genutzt werden.

12. Vorrichtung zum Ermitteln eines Schädigungsgrads von Haar (102), aufweisend:
eine UV-Lampe, insbesondere eine UV-LED, zum Belichten einer Haarprobe (102P) des Haars, in welchem vorzugsweise ein Fluoreszenzfarbstoff adsorbiert ist, mit UV-Licht (108);
eine Vorrichtung (110) zum Registrieren von Fluoreszenzlicht (112), welches von der Haarprobe emittiert wird; und
eine mobile Datenverarbeitungsvorrichtung (116) zum Ermitteln einer Fluoreszenzintensität anhand des registrierten Lichts und zum Ermitteln des Schädigungsgrads des Haars unter Einbeziehung der Fluoreszenzintensität,
wobei die Vorrichtung so eingerichtet ist, dass das Ermitteln des Schädigungsgrads durch eine Anwendung von einem mathematischen Modell erfolgt, wobei das mathematische Model mittels eines Verfahrens aus einer Gruppe von Verfahren erstellt wird, wobei die Gruppe von Verfahren aufweist:
lineare oder multi-lineare Regression,
polynome Regression,
neuronale-Netze-Verfahren,
Support Vector Machine-Verfahren; und
Entscheidungsbäume-Verfahren, wobei das mathematische Model mittels Vermessung von Standard-Haarproben, welche einen ermittelten Cysteinsäuregehalt aufweisen, erstellt wird, welches dann bei der Haarprobe anhand des registrierten Lichts und der daraus ermittelten Fluoreszenzintensität eine Berechnung eines Gehalts an Cysteinsäure, und damit des Schädigungsgrads, durchführt.

13. Verfahren zum Ermitteln eines nutzerspezifischen Mittels zur Haarbehandlung, aufweisend:
Ermitteln eines Schädigungsgrads von Haar (102) eines Nutzers gemäß einem der Ansprüche 1 bis 11;
computergestütztes Ermitteln von mittels einer Mehrzahl von Mitteln zur Haarbehandlung erzielbaren Behandlungsergebnissen mittels prädiktiver Analytik unter Einbeziehung des ermittelten Schädigungsgrads; und
Auswählen des nutzerspezifischen Mittels anhand der ermittelten Behandlungsergebnisse.

14. Verfahren gemäß Anspruch 13,
wobei das nutzerspezifische Mittel zur Haarbehandlung ein Haarpflegeprodukt oder ein Haarfärbeprodukt, ein Blondierungsprodukt, oder ein Stylingprodukt, beispielsweise ein Glättungsmittel, insbesondere einen Straightener oder Relaxer, oder ein Mittel für eine Dauerwelle, aufweist.

## Claims

1. A method for determining a degree of damage to hair (102), comprising:
while exposing a hair sample (102P) of the hair to UV light (108), recording light emitted by the hair sample;
determining a fluorescence intensity on the basis of the recorded light; and
determining the degree of damage to the hair, taking into account the fluorescence intensity of the hair sample,
wherein the degree of damage is determined using a mathematical model,
wherein the mathematical model is created by means of a method from a group of methods, wherein the group of methods comprises:
linear or multi-linear regression,
polynomial regression,
neural network methods,
support vector machine methods, and
decision tree methods, wherein the mathematical model is created by measuring standard hair samples which have a determined cysteic acid content, which model then calculates the cysteic acid content, and thus the degree of damage, in the hair sample on the basis of the recorded light and the fluorescence intensity determined therefrom.

2. The method according to claim 1, further comprising:
wetting the hair sample with a fluorescent dye solution (104); and
removing fluorescent dye solution not adsorbed in the hair sample;
wherein the recorded light is at least partially emitted by fluorescent dye adsorbed in the hair sample.

3. The method according to claim 1,
wherein determining the degree of damage comprises determining a relationship between a plurality of parameters influencing fluorescence intensity and the fluorescence intensity by means of predictive analysis.

4. The method according to claim 3,
wherein the plurality of parameters influencing fluorescence intensity includes a hair color and/or an ethnic origin of a hair type.

5. The method according to one of claims 1 to 4,
wherein recording light comprises taking a photo or detecting the light directly.

6. The method according to one of claims 1 to 5,
wherein light is recorded, a fluorescence intensity is determined and/or the degree of damage to the hair is determined by means of a mobile data processing device.

7. The method according to one of claims 2 to 6,
wherein the fluorescent dye solution comprises coumarin.

8. The method according to one of claims 1 to 7,
wherein the UV light has a wavelength in a range of approximately 315 nm to approximately 380 nm.

9. The method according to one of claims 2 to 8,
wherein wetting the hair sample with a fluorescent dye solution comprises wetting the hair for a predetermined period of time.

10. The method according to one of claims 1 to 9, further comprising:
before recording the light, producing defined conditions for exposing the hair sample to UV light and for recording the light.

11. The method according to one of claims 1 to 10,
wherein results of comparative measurements are used to determine the degree of damage to the hair.

12. A device for determining a degree of damage to hair (102), comprising:
a UV lamp, in particular a UV LED, for exposing a hair sample (102P) of the hair, in which a fluorescent dye is preferably adsorbed, to UV light (108);
a device (110) for recording fluorescent light (112) emitted by the hair sample; and
a mobile data processing device (116) for determining a fluorescence intensity on the basis of the recorded light and for determining the degree of damage to the hair, taking into account the fluorescence intensity,
wherein the device is designed such that the degree of damage is determined using a mathematical model,
wherein the mathematical model is created by means of a method from a group of methods, wherein the group of methods comprises:
linear or multi-linear regression,
polynomial regression,
neural network methods,
support vector machine methods, and
decision tree methods, wherein the mathematical model is created by measuring standard hair samples which have a determined cysteic acid content, which model then calculates the cysteic acid content and thus the degree of damage in the hair sample on the basis of the recorded light and the fluorescence intensity determined therefrom.

13. A method for determining a user-specific agent for hair treatment, comprising:
determining a degree of damage to the hair (102) of a user according to one of claims 1 to 11;
determining, in a computerized manner, treatment results that can be achieved by means of a plurality of agents for hair treatment by means of predictive analysis, taking into account the determined degree of damage; and
selecting the user-specific agent on the basis of the determined treatment results.

14. The method according to claim 13,
wherein the user-specific agent for hair treatment comprises a hair care product or a hair coloring product, a bleaching product, or a styling product, for example a straightening agent, in particular a straightener or relaxer, or an agent for a perm.

## Revendications

1. Procédé permettant de déterminer un degré de détérioration des cheveux (102), comprenant :
l'enregistrement, pendant l'exposition d'un échantillon (102P) des cheveux à la lumière UV (108), de la lumière émise par l'échantillon de cheveux ;
la détermination d'une intensité de fluorescence sur la base de la lumière enregistrée ; et
la détermination du degré de détérioration des cheveux en tenant compte de l'intensité de fluorescence de l'échantillon de cheveux,
le degré de détérioration étant déterminé par l'application d'un modèle mathématique,
le modèle mathématique étant élaboré au moyen d'un procédé sélectionné dans un groupe de procédés, le groupe de procédés comprenant :
une régression linéaire ou multilinéaire,
une régression polynomiale,
un procédé de réseau de neurones,
un procédé de machine à vecteur de support ; et
un procédé d'arbres de décision, le modèle mathématique étant élaboré par mesure d'échantillons de cheveux standards présentant une teneur en acide cystéique déterminée, lequel modèle mathématique effectue ensuite, sur l'échantillon de cheveux, un calcul d'une teneur en acide cystéique et, partant, du degré de détérioration sur la base de la lumière enregistrée et de l'intensité de fluorescence déterminée à partir de celle-ci.

2. Procédé selon la revendication 1, comprenant en outre :
le mouillage de l'échantillon de cheveux avec une solution de colorant fluorescent (104) ; et
l'élimination de la solution de colorant fluorescent non adsorbée sur l'échantillon de cheveux ;
la lumière enregistrée étant au moins partiellement émise par un colorant fluorescent adsorbé sur l'échantillon de cheveux.

3. Procédé selon la revendication 1,
dans lequel la détermination du degré de détérioration comprend la détermination d'une relation entre une pluralité de paramètres influençant l'intensité de fluorescence et l'intensité de fluorescence au moyen d'analyses prédictives.

4. Procédé selon la revendication 3,
dans lequel la pluralité de paramètres influençant l'intensité de fluorescence comprend une couleur de cheveux et/ou une origine ethnique d'un type de cheveux.

5. Procédé selon l'une des revendications 1 à 4,
dans lequel l'enregistrement de lumière comprend la génération d'une photo ou une détection directe de la lumière.

6. Procédé selon l'une des revendications 1 à 5,
dans lequel l'enregistrement de lumière, la détermination d'une intensité de fluorescence et/ou la détermination du degré de détérioration des cheveux sont effectués au moyen d'un dispositif de traitement de données mobile.

7. Procédé selon l'une des revendications 2 à 6,
dans lequel la solution de colorant fluorescent comprend de la coumarine.

8. Procédé selon l'une des revendications 1 à 7,
dans lequel la lumière UV présente une longueur d'onde dans une plage d'environ 315 nm à environ 380 nm.

9. Procédé selon l'une des revendications 2 à 8,
dans lequel le mouillage de l'échantillon de cheveux avec une solution de colorant fluorescent comprend le mouillage des cheveux pendant une durée prédéterminée.

10. Procédé selon l'une des revendications 1 à 9, comprenant en outre :
avant l'enregistrement de la lumière, la création de conditions définies pour l'exposition de l'échantillon de cheveux à la lumière UV et pour l'enregistrement de la lumière.

11. Procédé selon l'une des revendications 1 à 10,
dans lequel des résultats de mesures comparatives sont utilisés pour la détermination du degré de détérioration des cheveux.

12. Dispositif permettant de déterminer un degré de détérioration des cheveux (102), comprenant :
une lampe UV, en particulier une DEL UV, destinée à exposer un échantillon (102P) des cheveux à la lumière UV (108), un colorant fluorescent de préférence étant adsorbé sur l'échantillon de cheveux ;
un dispositif (110) destiné à enregistrer la lumière fluorescente (112) émise par l'échantillon de cheveux ; et
un dispositif de traitement de données mobile (116) destiné à déterminer une intensité de fluorescence sur la base de la lumière enregistrée et à déterminer le degré de détérioration des cheveux en tenant compte de l'intensité de fluorescence,
le dispositif étant configuré de telle sorte que le degré de détérioration est déterminé par l'application d'un modèle mathématique,
le modèle mathématique étant élaboré au moyen d'un procédé sélectionné dans un groupe de procédés, le groupe de procédés comprenant :
une régression linéaire ou multilinéaire,
une régression polynomiale,
un procédé de réseau de neurones,
un procédé de machine à vecteur de support ; et
un procédé d'arbres de décision, le modèle mathématique étant élaboré par mesure d'échantillons de cheveux standards présentant une teneur en acide cystéique déterminée, lequel modèle mathématique effectue ensuite, sur l'échantillon de cheveux, un calcul d'une teneur en acide cystéique et, partant, du degré de détérioration sur la base de la lumière enregistrée et de l'intensité de fluorescence déterminée à partir de celle-ci.

13. Procédé permettant de déterminer un agent spécifique à l'utilisateur destiné au traitement des cheveux, comprenant : la détermination d'un degré de détérioration des cheveux (102) d'un utilisateur selon l'une des revendications 1 à 11 ;
la détermination assistée par ordinateur de résultats de traitement pouvant être obtenus au moyen d'une pluralité de moyens destinés au traitement des cheveux en effectuant des analyses prédictives en tenant compte du degré de détérioration déterminé ; et
la sélection de l'agent spécifique à l'utilisateur sur la base des résultats de traitement déterminés.

14. Procédé selon la revendication 13,
dans lequel l'agent spécifique à l'utilisateur destiné au traitement des cheveux comprend un produit de soin des cheveux ou un produit de coloration des cheveux, un produit de décoloration ou un produit de coiffage, par exemple un agent de lissage, en particulier un lisseur ou un défrisant, ou un agent pour une permanente.
